# FASCICULE DE BREVET EUROPEEN

(11) **EP 3 066 521 B1**
(45) Date de publication et mention de la délivrance du brevet: **20.09.2023**
(21) Numéro de dépôt: 14825381.8
(22) Date de dépôt: 28.10.2014
(51) Int. Cl.: G02C 7/02, G02C 7/06, A61B 3/113, A61B 3/00

(54) **METHODE DE DETERMINATION D'AU MOINS UN PARAMETRE DE CONCEPTION OPTIQUE D'UNE LENTILLE OPHTALMIQUE PROGRESSIVE**
VERFAHREN ZUR BESTIMMUNG MINDESTENS EINES OPTISCHEN DESIGNPARAMETERS FÜR EINE PROGRESSIVE KONTAKTLINSE
METHOD FOR DETERMINING AT LEAST ONE OPTICAL DESIGN PARAMETER FOR A PROGRESSIVE OPHTHALMIC LENS

(30) Priorité: 08.11.2013 FR 1360991
(43) Date de publication de la demande: 14.09.2016
(73) Titulaire: Essilor International, 94220 Charenton-le-Pont (FR)
(72) Inventeur: BARANTON, Konogan, 94227 Charenton-Le-Pont (FR); BONNIN, Thierry, 94227 Charenton-Le-Pont (FR); WIERZBICKI, Juliette, 94227 Charenton-Le-Pont (FR); HADDADI, Ahmed, 94227 Charenton-Le-Pont (FR); MARIE, Sarah, 94227 Charenton-Le-Pont (FR); DROBE, Bjorn, Singapour 339346 (SG)
(74) Mandataire: Jacobacci Coralis Harle
(86) Numéro de dépôt international: PCT/FR2014/052737
(87) Numéro de publication internationale: WO 2015/067876

(56) Documents cités:
- EP-A1- 1 747 750
- WO-A1-2012/038676
- FR-A1- 2 894 688
- US-B1- 6 286 957

## Description

### DOMAINE TECHNIQUE AUQUEL SE RAPPORTE L'INVENTION

La présente invention concerne une méthode de détermination d'un paramètre de conception optique d'une lentille ophtalmique progressive.

### ARRIERE-PLAN TECHNOLOGIQUE

Les lentilles ophtalmiques progressives permettent au porteur de bénéficier d'une compensation de puissance optique adaptée pour différentes distances de vision sans changer de lunettes. Elles peuvent également corriger d'autres défauts visuels, comme par exemple l'astigmatisme.

Une lentille ophtalmique progressive présente une puissance variable sur la surface de la lentille.

Il est par exemple prévu une première zone de vision pour la vision de loin présentant une première valeur de puissance moyenne, une seconde zone de vision pour la vision de près présentant une seconde valeur de puissance moyenne et, entre ces deux zones, une troisième zone de vision pour la vision intermédiaire, dont la courbure varie progressivement et qui est appelée couloir de progression.

La différence entre les première et seconde valeurs de puissance moyenne est égale à l'addition de puissance de la lentille.

La croix de montage est un point de référence pour le positionnement de la lentille devant l'oeil d'un porteur dont la position est prédéfinie par le fabriquant de la lentille.

La première zone pour la vision de loin est centrée sur un point de référence pour la vision de loin dont la position est prédéterminée pour une lentille donnée par le fabriquant.

La deuxième zone pour la vision de près est centrée sur un point de référence pour la vision de près dont la position est prédéterminée pour une lentille donnée par le fabriquant.

La première zone pour la vision de loin et la deuxième zone pour la vision de près sont séparées par une distance appelée longueur de progression.

La longueur de progression peut être définie comme la distance verticale entre la croix de montage et la position du point de référence en vision de près défini par le fabricant du verre.

Les directions horizontales et verticales de la lentille sont définies en fonction de la position de la lentille en conditions d'utilisation par le porteur, dans la monture choisie.

La longueur de progression de la lentille doit être ajustée en fonction de la hauteur de montage de la lentille ophtalmique.

La hauteur de montage de la lentille ophtalmique correspond à la hauteur, par rapport au bord inférieur du cercle de la monture, de la projection de la pupille du porteur ayant une direction de regard primaire prédéterminée sur un plan moyen de ce cercle de la monture choisie, correspondant à un plan moyen de la lentille ophtalmique une fois montée dans ladite monture.

Cette direction de regard primaire prédéterminée correspond à la direction de regard du porteur dans des conditions de vision de loin.

La longueur de progression de la lentille est ajustée de sorte que la deuxième zone pour la vision de près de la lentille soit inclue dans la lentille une fois détourée et positionnée dans la monture choisie.

En outre, le positionnement de la deuxième zone utilisée pour la vision de près peut être réalisé en fonction des habitudes visuelles du porteur.

Habituellement, le choix de la longueur de progression est fait par l'opticien à partir de critères subjectifs tels que la posture du porteur ou le retour d'expérience de celui-ci sur son équipement passé.

On connaît également du document US8297752 une méthode pour déterminer la distance de progression de la lentille selon laquelle on détermine un unique point de vision de loin du porteur et un unique point de vision de près du porteur sur la lentille ophtalmique et on en déduit la longueur de progression correspondante. Une lentille ophtalmique adaptée au porteur peut ainsi être sélectionnée. WO2012/038676 A1 est un document similaire.

Cependant, il n'est pas certain, en appliquant cette méthode, qu'une partie suffisante pour une utilisation confortable de la deuxième zone pour la vision de près utilisée par le porteur soit inclue dans la lentille ophtalmique une fois celle-ci détourée et montée dans la monture choisie par le porteur. De manière générale, la détermination précise de la longueur de progression implique en effet une détermination précise de la position des première et deuxième zones de vision. Le positionnement précis de la monture sur le visage du porteur, résultant par exemple de la géométrie de la monture et de l'habillage de cette monture sur la tête du porteur, influence directement la position des première et deuxième zones de vision pour un porteur déterminé. Ces paramètres ne sont pas pris en compte dans l'état de la technique. On connaît également du document EP1747750 une méthode de personnalisation d'une paire de lunettes en fonction du comportement visuel du porteur, en particulier les mouvements relatifs de ses yeux par rapport à sa tête et la durée pendant laquelle ses yeux gardent une orientation fixe par rapport à la tête du porteur.

### OBJET DE L'INVENTION

Afin de remédier à l'inconvénient précité de l'état de la technique, la présente invention propose une méthode de détermination d'au moins un paramètre de conception optique d'une lentille ophtalmique progressive destinée à équiper une monture choisie par un porteur, permettant de prendre en compte à la fois le comportement visuel particulier du porteur et les dimensions de la monture choisie par ce porteur afin de définir les paramètres de conception optique (design optique) de la lentille ophtalmique les mieux adaptés à la fois au porteur et à la monture.

Plus particulièrement, on propose selon l'invention une telle méthode comprenant les étapes décrites dans la revendication 1 et un système comprenant les caractéristiques décrites dans la revendication 15. comprenant les étapes décrites dans la revendication 1.

D'autres caractéristiques non limitatives et avantageuses de la méthode conforme à l'invention sont listées dans les revendications 1 à 14. oeuvre de méthode, telle que décrite dans la revendication

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique d'un mode de réalisation de la détermination de différentes directions de regard de l'œil d'un porteur,
- la figure 2 est une vue schématique de la répartition des points d'intersection de différentes directions de regard avec un plan moyen d'un cercle de la monture,
- la figure 3 est une vue schématique de face d'un premier exemple de lentille ophtalmique dans laquelle la zone d'usure en vision de près est située à une première position par rapport à un point de référence de vision de loin,
- la figure 4 est une vue schématique de face d'un deuxième exemple de lentille ophtalmique dans laquelle la zone d'usure en vision de près est située à une deuxième position par rapport au point de référence de vision de loin.

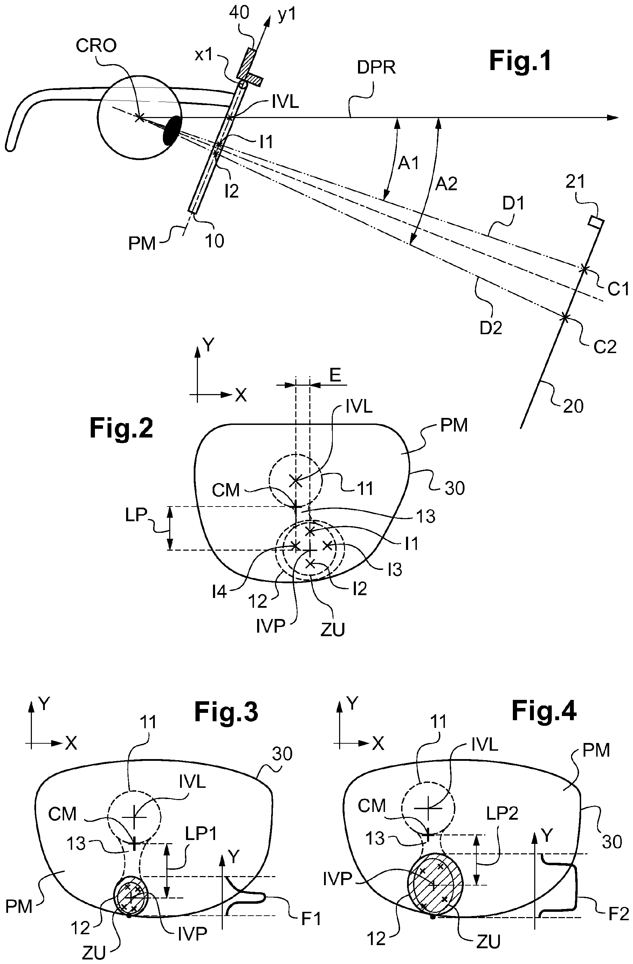

On notera que les éléments identiques ou correspondants des différentes lentilles ophtalmiques représentées en projection dans le plan moyen de la monture sur les figures 1 à 4 seront référencés par les mêmes signes.

La lentille 30 représentée sur les figures 2 à 4 est une lentille ophtalmique à addition progressive de puissance sphérique, ou lentille zone de vision 11 dont la puissance sphérique est adaptée pour la vision de loin du porteur en fonction de ses besoins de correction visuelle et, dans sa partie inférieure, une seconde zone de vision 12 dont la puissance sphérique est adaptée pour la vision de près de ce porteur.

Les figures 2 à 4 montrent la projection de cette lentille 30 dans le plan moyen PM du cercle correspondant de la monture 10 choisie.

Dans la suite, on nommera la première zone 11 pour la vision de loin « zone de vision de loin 11 » et la deuxième zone 12 pour la vision de près « zone de vision de près 12 ».

Entre les zones de vision de loin 11 et de vision de près 12 se trouve, de façon connue, une troisième zone de vision 13 qui est adaptée pour la vision à distance intermédiaire.

La zone de vision de loin 11 entoure un point de référence de la vision de loin IVL, tandis que la zone de vision de près 12 entoure un point de référence de la vision de près IVP.

Au point de référence pour la vision de loin IVL, la lentille 30 présente une première puissance sphérique prédéterminée adaptée pour la vision de loin du porteur, tandis que, au point de référence pour la vision de près IVP, elle a une seconde puissance sphérique prédéterminée adaptée pour la vision de près du porteur.

La puissance de la lentille varie, de préférence continument, entre lesdits points de référence pour la vision de loin IVL et pour la vision de près IVP, le long d'une courbe appelée « courbe méridienne principale de progression » qui passe par ces deux points. Cette courbe méridienne principale de progression traverse ces trois zones VL, VI et VP suivant une direction globalement verticale.

Dans le cadre de la présente description, on admettra les définitions suivantes.

Conformément à la norme ISO 13666:2012, on nomme point de montage le point situé sur la surface avant d'une lentille que le fabricant considère comme point de référence pour le positionnement du verre devant l'oeil.

La position du point de montage sur la lentille est prédéterminée et connue.

La hauteur de montage correspond alors à la distance verticale qui sépare le point de montage de la tangente horizontale passant par le point inférieur de la périphérie de la lentille.

Dans la suite de la description, on définit une zone d'usure ZU d'un verre comme étant une zone de l'espace représentative d'une distribution statistique d'un ensemble de points sur le verre par lesquels passe le regard du porteur lors d'une tâche particulière de vision ou pour une utilisation à une distance de travail prédéterminée. La zone d'usure ZU peut être définie de manière équivalente soit spatialement, par une distribution statistique de points l1 l2sur la lentille ophtalmique ou sur un autre plan de projection lié à la lentille ophtalmique ou au cercle de la monture correspondant, soit vectoriellement, par une distribution statistique de directions du regard A1, A2. De manière alternative et simplifiée, la zone d'usure ZU peut aussi être définie de manière tabulée par une distribution statistique d'angles d'abaissement du regard A1, A2 dans le plan sagittal du porteur.

On définit la longueur de progression LP de la lentille ophtalmique comme la distance verticale entre la croix de montage CM et la position du point de référence en vision de près IVP défini par le fabricant du verre (figure 2).

La croix de montage CM est un point de référence pour le positionnement de la lentille devant l'oeil d'un porteur dont la position est prédéfinie par le fabriquant de la lentille.

D'autres définitions peuvent être adoptées pour la longueur de progression. Elle peut être exprimée par rapport au point de référence du prisme ou au point de référence de vision de loin IVL plutôt que par rapport à la croix de montage. Comme les positions respectives de ces points sont par ailleurs aussi données par le fabricant cette définition est équivalente à la précédente.

Quelle que soit la définition considérée de la longueur de progression, la méthode selon l'invention reste la même.

La surface moyenne de la lentille ophtalmique est définie comme la surface équidistante en tout point des faces avant et arrière de la lentille.

La direction horizontale est considérée comme la perpendiculaire à la direction verticale, suivant par exemple un fil à plomb.

La direction du regard est une droite appartenant à un plan contenant le point fixé du regard par le porteur et les centres de rotation des yeux.

Pour un oeil en particulier, la direction de regard est définie comme la droite reliant le point fixé du regard par le porteur et le centre de rotation de cet oeil.

En vision de loin, avec un point de visé droit devant à l'infini, la direction du regard est horizontale. Cette direction du regard correspond à la direction de regard primaire DPR (figure 1).

La lentille ophtalmique progressive se définit notamment par deux grandeurs optiques principales :
- l'addition égale à la variation de puissance entre le point de référence en vision de loin IVL et le point de référence en vision de près IVP,
- une « puissance nominale » égale à la puissance audit point de référence pour la vision de loin IVL.

On définit l'inset E de la lentille comme le décalage horizontal entre le point de référence pour la vision de loin IVL et le point de référence pour la vision de près IVP. L'inset E est aussi appelé « déport interne ».

Afin de fournir au porteur le plus grand confort visuel possible, il est nécessaire de positionner précisément les zones de vision de loin et de près des deux lentilles ophtalmiques destinées à équiper ce porteur, de manière à ce que celui-ci regarde à travers la zone de vision de loin 11 lorsqu'il regarde au loin et à travers la zone de vision de près 12 lorsqu'il effectue une tâche visuelle en vision de près.

La position relative et les dimensions de ces deux zones de vision de loin et de près dépendent ainsi des paramètres géométrico-physionomiques du porteur, comme son écart inter-pupillaire par exemple, et de son comportement visuel. Elles dépendent également des caractéristiques géométriques de la monture choisie par le porteur, notamment de la hauteur des cercles de la monture, de la base de celle-ci ou de l'angle pantoscopique de la monture portée par le porteur.

Grâce à la méthode selon l'invention, il est possible de déterminer la longueur de progression de chaque lentille ophtalmique progressive, en fonction du comportement visuel du porteur.

Cette méthode permet en particulier d'évaluer le comportement visuel du porteur dans une posture naturelle lors d'une tâche visuelle en vision de près et de prendre en compte les paramètres géométrico-morphologiques du porteur ainsi que la géométrie de la monture choisie.

On entend par posture naturelle, la posture qui est adoptée habituellement par le porteur en l'absence de toute contrainte.

Ce paramètre de conception optique peut être en particulier lié à la position relative des zones de vision de loin 11 et de vision de près 12.

Il s'agit par exemple de la longueur de progression LP ; LP1 ; LP2 de la lentille ophtalmique ou de l'inset E (figures 2 à 4).

Plus précisément, selon l'invention, on effectue les étapes suivantes :
a) on place le porteur dans une situation dans laquelle il effectue une tâche visuelle à une première distance de travail,
b) on détermine, lors de cette tâche visuelle, au moins deux directions D1, D2 du regard du porteur à cette première distance de travail, dans un référentiel (x1, y1) lié à la tête du porteur,
c) on détermine une position relative d'une surface PM ou d'une ligne liée à ladite monture 10 ou à une lentille ophtalmique 30 destinée à équiper ladite monture 10 dans ce référentiel (x1, y1) lié à la tête du porteur,
d) on détermine, pour chaque direction D1, D2 du regard déterminée à l'étape b), l'intersection entre cette direction du regard à la première distance de travail et ladite surface ou ladite ligne, de manière à établir une cartographie de ces points d'intersection I1, I2, I3, I4sur cette surface, et
e) on déduit de cette cartographie la longueur de progression recherchée.

### Etape a)

On place le porteur dans une situation dans laquelle il effectue une tâche ayant au moins une composante visuelle à une première distance de travail en vision de près.

La distance de travail est définie comme la distance entre les yeux du porteur et le point fixé du regard par le porteur.

Dans l'exemple décrit plus particulièrement ici, la première distance de travail est comprise entre 20 et 60 centimètres, par exemple égale à 40 centimètres. Cette première distance de travail correspond alors à un travail en vision de près.

En variante, on peut envisager de mettre en oeuvre la méthode selon un exemple non couvert par l'invention pour une première distance de travail comprise entre 60 centimètres et 1,5 mètre, ce qui correspond à un travail en vision intermédiaire, ou entre 1,5 mètre et l'infini, ce qui correspond à un travail en vision de loin.

Plus précisément, ici, à l'étape a), l'opérateur demande au porteur d'effectuer de préférence une tâche choisie parmi les tâches suivantes :
- tâche de lecture,
- tâche d'écriture,
- tâche interactive,
- tâche d'observation.

Pour l'exécution de cette tâche, on présente au porteur un support 20 (figure 1) qu'il peut tenir entre ses mains, et placer de la manière qu'il souhaite par rapport à sa tête.

Ce support 20 est de préférence un support plan comportant une partie d'affichage numérique. Il s'agit par exemple de préférence d'une tablette tactile.

Ce support 20 comporte de manière générale une cible C1, C2 que le porteur doit suivre du regard pendant la tâche qui lui est assignée.

Par exemple, pour une tâche de lecture, la cible est constituée par chaque mot du texte affiché sur le support.

Il peut s'agir par exemple d'un texte affiché sur un écran dans une couleur noire avec, dans une autre couleur, en surbrillance ou souligné, le mot à lire, qui se déplace le long du texte.

Le porteur lit à chaque instant le mot signalé par la couleur différente du texte, la surbrillance ou le soulignement.

Pour une tâche d'écriture, il s'agit cette fois des mots à écrire.

La tâche d'écriture est réalisée par exemple en utilisant un stylet sur la tablette tactile constituant le support 20. Il est alors possible d'identifier directement l'endroit que regarde le porteur pendant qu'il écrit comme étant le point de contact entre l'extrémité du stylet et l'écran actif de la tablette tactile.

On entend par tâche interactive une tâche du type jeu interactif, dans lequel la cible C1, C2 est constituée d'une image que le porteur doit suivre des yeux. L'interactivité est liée au fait que le porteur doit agir, par exemple, cliquer sur le support, en fonction de différentes caractéristiques de la cible : position, orientation, contraste...

On peut par exemple envisager que la cible C1, C2 soit constituée par l'image d'une flèche et que le porteur clique sur le support 20 à droite ou à gauche suivant la direction de la flèche.

La cible C1, C2 peut également se déplacer sur la partie d'affichage du support, en ligne droite, et le porteur doit cliquer avant qu'elle ne touche le bord de cette partie d'affichage.

La cible C1, C2 peut encore être constituée d'une image lumineuse clignotant dont le contraste augmente progressivement. Le porteur clique sur le support dès qu'il voit apparaître la cible.

On entend par tâche d'observation une tâche au cours de laquelle le porteur suit des yeux la cible C1, C2, par exemple une image, sans interagir avec elle.

De manière générale, quelle que soit la tâche considérée, on peut envisager de réaliser une étape de réglage préalable à la réalisation de la tâche elle-même afin d'ajuster les dimensions d'affichage de la cible C1, C2 sur le support 20 en fonction de l'acuité du porteur.

On s'assure ainsi que le porteur puisse voir distinctement la cible pendant la tâche à réaliser, et ce quel que soit l'équipement dont il dispose au moment d'effectuer cette tâche.

De préférence, à l'étape a), on place sur la tête du porteur la monture 10 qu'il a choisi, sans aucune lentille montée à l'intérieur (figure 1).

On peut aussi envisager de placer sur la tête du porteur une monture autre que celle choisie, présentant de préférence des dimensions supérieures à celle choisie de manière à ce que le regard du porteur ne soit pas contraint par les bords de la monture.

On peut également envisager de placer sur la tête du porteur la monture choisie avec des lentilles de présentation, ne présentant aucune puissance, ou avec des lentilles correctrices, par exemple des lentilles similaires à celles que le porteur utilise couramment.

La monture 10 est de préférence équipée d'un système de repérage 40 destiné à permettre la détermination de la position de la tête du porteur dans l'espace à partir d'une image capturée de la tête du porteur équipée du système de repérage. Ce système de repérage 40 est décrit en détails dans le document FR2914173, page 7, ligne 5 à page 10, ligne 8. Il ne sera donc pas décrit plus en détail ici.

Ce système de repérage 40 présente des caractéristiques géométriques prédéterminées, qui permettent, à partir d'une image capturée de la tête du porteur sur laquelle apparaît ce système de repérage, de déterminer la position et l'orientation de la tête du porteur dans l'espace, dans le référentiel lié au dispositif de capture d'image. Ce système de repérage 40 permet donc de déterminer la position et l'orientation du référentiel lié à la tête du porteur dans le référentiel lié au dispositif de capture d'image.

On peut également envisager que la monture elle-même, placée sur la tête du porteur, joue le rôle du système de repérage.

On peut également envisager de ne placer sur la tête du porteur ni monture, ni lentille. Dans ce cas, la tête du porteur peut être directement équipée du système de repérage.

### Etape b)

Afin de permettre la détermination des directions du regard du porteur dans la première distance de travail pendant que celui-ci exécute la tâche visuelle qui lui a été assignée, le support 20 comporte au moins un dispositif de capture d'image 21.

Il s'agit de préférence d'une caméra pour réaliser une acquisition vidéo du porteur pendant la tâche visuelle.

La position de la cible C1, C2 que le porteur suit des yeux pendant la tâche visuelle est connue à tout instant par rapport au support 20. Elle est donc connue dans le référentiel lié au dispositif de capture d'image 21.

Ainsi, grâce à cet agencement, la position de la cible C1, C2 que le porteur fixe du regard au moment de la capture d'image est connue dans le référentiel lié au dispositif de capture d'image et donc dans le référentiel lié à la tête du porteur.

A l'étape b), on réalise alors, à l'aide de ce dispositif de capture d'image 21, une capture d'image de la tête du porteur pour différentes positions de la cible C1, C2 correspondant à différentes directions D1, D2 de regard à la première distance de travail.

Le dispositif de capture d'image et les moyens d'affichage de la partie d'affichage du support 20 sont de préférence synchronisés de manière à ce que la capture d'une image soit déclenchée lorsque la cible C1 est dans une position prédéterminée, ou de manière à ce que la position de la cible C1 dans le référentiel lié au dispositif de capture d'image au moment de la capture d'image soit mis en mémoire et associée à l'image capturée à ce moment.

Comme évoqué précédemment, l'acquisition d'image peut être réalisée via l'enregistrement d'une vidéo.

On peut également envisager, dans le cas d'une tâche interactive, que le dispositif de capture d'image 21 soit configuré pour enregistrer une image de la tête du porteur uniquement lorsque celui-ci clique sur le support 20.

Les images de la tête du porteur capturées sont transmises à une unité de traitement informatique qui peut être intégrée au support ou déportée.

Les images capturées peuvent être traitées en temps réel ou après la capture de toutes les images.

A partir de ces images capturées, l'unité de traitement détermine la position du centre de rotation CRO d'au moins un oeil du porteur dans le référentiel lié à la tête du porteur.

Le principe de cette détermination est connu en soi et exposé par exemple dans le document FR2914173, dont un équivalent en anglais est le document US20100128220.

A titre d'exemple, il est possible d'identifier les images d'un point remarquable de l'oeil du porteur, par exemple de la pupille de l'oeil du porteur sur deux images capturées alors que le porteur fixe des yeux des cibles C1, C2 présentant une position différente par rapport au dispositif de capture d'image. La position de la pupille de l'oeil par rapport au dispositif de capture d'image est ensuite déterminée en fonction des caractéristiques géométriques de l'image du système de repérage 40 sur ces deux images. Ces caractéristiques géométriques donnent accès à un facteur d'échelle de l'image et aux angles de rotation de la tête du porteur par rapport au dispositif de capture d'image 21.

La position de la cible C1, C2 fixée du regard pendant les deux captures d'image étant connue par rapport au dispositif de capture d'image, on en déduit la position du centre de rotation de l'oeil CRO comme étant l'intersection des droites passant par la cible et la pupille de l'oeil pour chaque image capturée.

On peut également déterminer pour chaque direction de regard la posture de la tête du porteur correspondante, c'est-à-dire sa position et son orientation dans le référentiel lié au dispositif de capture d'image. Un couple de données associant la posture de la tête et la direction de regard est mis en mémoire dans un fichier.

Selon un premier mode de réalisation de la méthode suivant l'invention, l'unité de traitement informatique en déduit la direction du regard D1, D2 à la première distance de travail du porteur dans le référentiel lié à la tête du porteur lors de chaque capture d'image comme étant la droite reliant ce centre de rotation CRO avec la cible C1, C2 dans sa position correspondante pendant la capture d'image.

En variante, la position du centre de rotation de l'oeil CRO du porteur dans le référentiel de la tête du porteur peut être prédéterminée. Pour cela, les étapes de captures et de traitement d'images peuvent être réalisées préalablement à la mise en oeuvre du procédé selon l'invention, éventuellement dans un lieu ou grâce à un appareil de mesure différent.

Dans ce cas, la position du centre de rotation de l'oeil CRO du porteur peut être mise en mémoire manuellement ou transmise directement à l'unité de traitement informatique.

Selon un deuxième mode de réalisation, à l'étape b), on détermine la position du centre de rotation de l'oeil CRO en fonction d'une position moyenne prédéterminée de ce centre de rotation, par exemple par rapport à la face arrière de la lentille ophtalmique. A cet effet, on considère par exemple que le centre de rotation est situé à une distance moyenne égale à 27 millimètres de la face arrière de la lentille ophtalmique.

En variante, on peut également mesurer une position de référence des centres de rotation des yeux dans le référentiel lié à la tête du porteur dans une étape préliminaire, par une méthode connue en elle-même, enregistrer et transmettre cette position de référence à l'unité de traitement informatique.

Selon un troisième mode de réalisation, à l'étape b), on capture chaque image du porteur lorsque la cible C1, C2 se trouve sur l'axe optique du dispositif de capture d'image. On envisage ici un agencement alternatif dans lequel le dispositif de capture d'image est disposé derrière la partie d'affichage du support 20. La tâche visuelle du porteur est alors de préférence une tâche d'observation pendant laquelle il suit des yeux la cible C1, C2. Le mouvement de cette cible est paramétré de manière à ce qu'elle repasse à plusieurs reprises devant le dispositif de capture d'image situé en arrière, et la capture d'image est déclenchée aux instants correspondants à ces passages.

Il peut s'agir par exemple de tâches de lecture ou d'écriture telles que décrites précédemment, au cours desquelles la cible est constituée soit d'un mot repéré par une couleur, une surbrillance ou un soulignement (tâche de lecture), soit du point de contact du stylet sur la tablette pendant l'écriture d'un mot (tâche d'écriture).

La position du point de visée fixé des yeux par le porteur est ainsi précisément connue.

On peut alors déterminer directement la position du point d'intersection de la direction de regard et de la lentille ophtalmique ou du plan moyen du cercle de la monture comme étant la position de l'image de la pupille de l'oeil du porteur par rapport à l'image de la lentille ophtalmique sur l'image capturée. On détermine par exemple la position de l'image de la pupille par rapport à l'image des cercles de la monture. Ce faisant, la direction du regard est déterminée à l'étape b) comme étant la droite reliant la pupille de l'oeil du porteur et la pupille du dispositif de capture d'image, qui passe ici par la cible. La surface liée à la monture ou à la lentille ophtalmique est déterminée à l'étape c) comme étant confondue avec le plan de capture d'image. Le centre de rotation de l'oeil n'est pas déterminé.

Alternativement, on peut envisager que le dispositif de capture d'image se déplace de manière à ce que la cible reste toujours sur son axe optique.

On détermine alors la position des pupilles des yeux du porteur par rapport au système de repérage 40 ou à la monture 10, à partir des images capturées de la tête du porteur, pour en déduire la direction du regard du porteur à la première distance de travail comme la droite reliant la pupille à la cible C1, C2. Le centre de rotation de l'oeil n'est pas déterminé.

Selon une variante de chacun des modes de réalisation évoqué précédemment, dans le cas où le porteur est équipé avec une monture et des lentilles ophtalmiques correctrices, c'est-à-dire présentant une puissance prédéterminée, à l'étape b), l'unité de traitement est de préférence programmée pour déterminer la position du centre de rotation de l'oeil du porteur dans le référentiel lié à la tête du porteur et pour en déduire la direction du regard à la première distance de travail comme étant le chemin optique reliant le centre de rotation de l'oeil et la cible en tenant compte de la déviation prismatique de la lentille ophtalmique correctrice correspondante.

Il s'agit alors de tenir compte d'une part de la déviation prismatique lors de la détermination de la position du centre de rotation de l'oeil et, d'autre part, de la détermination de la direction de regard à la première distance de travail.

Cette direction de regard est typiquement déterminée par un calcul de tracé de rayon lumineux entre le centre de rotation de l'oeil et la cible, prenant en compte cette déviation prismatique. Afin de faire ce calcul de tracé de rayon lumineux, la position de la lentille ophtalmique correctrice par rapport à la tête du porteur est prise en compte.

### Etape c)

Afin de déterminer la position du point d'intersection I1, I2, I3, I4 de la direction du regard D1, D2 à la première distance de travail du porteur déterminée à l'étape b) et d'une surface PM ou d'une ligne liée à ladite monture ou à une lentille ophtalmique équipant ladite monture à l'étape d), on détermine la position de cette surface ou de cette ligne dans un référentiel lié à la tête du porteur.

Tout d'abord, la surface ou la ligne considérée peut être définie de différentes manières.

De manière préférentielle, la surface ou la ligne considérée est liée à au moins un des cercles de la monture. Il s'agit ainsi d'une surface ou d'une ligne représentative de la position de ce cercle de la monture lorsqu'elle est disposée sur la tête du porteur.

On entend ici par « liée à au moins un des cercles de la monture » le fait que cette surface ou cette ligne constitue un modèle surfacique ou linéaire d'au moins un des cercles de la monture.

Comme décrit en référence à l'étape a), on considère plus particulièrement dans l'exemple décrit ici que le porteur est équipé pour la mise en oeuvre de cette méthode de la monture 10 qu'il a choisi, sans lentille ophtalmique montée à l'intérieur des cercles de cette monture.

Dans cet exemple, la surface considérée à l'étape c) sera de préférence le plan moyen PM du cercle de la monture 10 disposé devant l'oeil considéré du porteur.

Cependant, en variante, il peut s'agir du plan moyen des deux cercles de la monture.

Il peut également s'agir d'un plan lié au système de repérage 40 disposé sur la monture 10.

En variante encore, il peut s'agir de la surface moyenne de la lentille ophtalmique destinée à être montée dans le cercle de la monture 10 ou de la face avant ou arrière de cette lentille ophtalmique.

La surface considérée peut donc être plane, comme cela est le cas du plan moyen du ou des cercles de la monture ou de la lentille, ou courbe, comme cela est le cas de la surface moyenne de la lentille ophtalmique correspondante.

Lorsque l'on considère une ligne liée à la monture ou à la lentille ophtalmique équipant cette monture, ladite ligne peut être une ligne droite ou courbe.

Il peut s'agir en particulier de l'intersection entre, d'une part, un plan parallèle au plan sagittale de la tête du porteur ou un plan appelé « plan de Francfort » et, d'autre part, la surface liée à la monture ou à la lentille ophtalmique telle que définie précédemment.

Le plan de Francfort PF de la tête du porteur est défini comme le plan passant par les points orbitaires inférieurs OR et le porion PO du porteur, le porion étant le point du crâne le plus élevé du conduit auditif, qui correspond au tragion de l'oreille.

Lorsque le porteur est en posture naturelle, ce plan de Francfort PF est sensiblement horizontal.

C'est le cas par exemple lorsque le porteur est dans une configuration assise ou debout dans laquelle sa tête est droite et qu'il regarde droit devant lui, au loin, c'est-à-dire de préférence à l'horizon. On dit également que le porteur prend une position orthostatique, ou une position dans laquelle il réalise un minimum d'efforts.

On définit un plan sagittal de la tête du porteur comme étant le plan perpendiculaire au plan de Francfort passant par la médiatrice des deux yeux. La médiatrice des yeux est l'axe passant au milieu du segment défini par les centres de rotation des deux yeux et parallèle au plan de Francfort.

La position de la surface considérée, ici par exemple le plan moyen PM du cercle de la monture, ou de la ligne peut être soit déterminée à partir des images capturées à l'étape b), simultanément, avant ou après le traitement de ces images pour déterminer la direction de regard du porteur à la première distance de travail, soit être prédéterminée préalablement à la réalisation de l'étape d).

Dans un exemple illustrant le premier cas, la position du plan moyen PM du cercle de la monture 10 dans le référentiel lié à la tête du porteur est déterminée en fonction de l'image de la monture et/ou de la lentille ophtalmique sur l'image capturée et en fonction de l'image du système de repérage 40 correspondante. Dans ce cas, l'unité de traitement informatique en déduit également la position de la surface considérée dans le référentiel du dispositif de capture d'image, par les mêmes moyens.

Dans un exemple illustrant le deuxième cas, l'opérateur réalise, préalablement à la réalisation de l'étape d), et de préférence, avant la mise en oeuvre des étapes a) et b), une étape de calibrage, dans laquelle il détermine la position de ce plan moyen PM par toute méthode connue de l'homme du métier. Il est possible d'envisager par exemple que cette position soit déterminée à partir d'une image capturée de la tête du porteur équipé de la monture de profil.

On peut également envisager que cette position de la surface considérée - plan moyen du ou des cercles de la monture, surface moyenne ou face de la lentille ophtalmique - ou de la ligne considérée, dans le référentiel lié à la tête du porteur soit issue d'une base de données prédéterminée comportant des positions moyennes de la surface ou de la ligne considérée. Ceci s'applique en particulier au cas où aucune monture ni lentille ophtalmique n'est placé sur la tête du porteur à l'étape a). On réalise alors dans l'étape c) un habillage virtuel de la tête du porteur, en replaçant virtuellement la monture ou la lentille ophtalmique sur la tête du porteur grâce aux informations issues de la base de données.

Lorsqu'il s'agit de déterminer la position de ladite ligne liée à la monture ou à la lentille ophtalmique, il est plus particulièrement possible de réaliser cette étape à partir d'une image de profil du porteur équipé de la monture. Cette capture d'image de profil est réalisée de préférence préalablement à la mise en oeuvre des autres étapes décrites ici. La ligne considérée correspond alors par exemple à l'intersection entre le plan moyen des cercles de la monture et le plan de capture d'image, qui est ici parallèle au plan sagittal de la tête du porteur.

Cette ligne est alors inclinée, dans la posture naturelle du porteur, d'un angle par rapport à la direction verticale égal à l'angle pantoscopique de la monture.

Il est également possible de déterminer cette ligne liée à la monture ou à la lentille ophtalmique comme étant l'intersection entre l'une des surfaces définie précédemment et le plan de Francfort de la tête du porteur. La position du plan de Francfort est prédéterminée ou déterminée à partir des captures d'image de face et/ou de profil du porteur.

On peut enfin envisager que l'orientation de la surface ou de la ligne considérée soit connue dans le référentiel de la tête du porteur, après une étape de calibration, par exemple déterminée à partir d'une image capturée de profil de la tête du porteur équipé de la monture.

Afin de replacer précisément cette surface ou cette ligne par rapport à la tête du porteur pendant la tâche visuelle du porteur, on détermine alors à l'étape c) la position d'au moins un point de référence associé à la monture ou à la lentille ophtalmique dans le référentiel de la tête du porteur à partir des images capturées à l'étape b). La position de la surface ou de la ligne considérée est ainsi déterminée avec précision pour chaque image capturée.

En variante, la surface ou la ligne considérée peut présenter un décalage, c'est-à-dire s'étendre dans un plan ou dans une direction parallèle à l'une des surfaces ou à l'une des lignes définies précédemment.

### Etape d)

A l'étape d), l'unité de traitement informatique est programmée pour déterminer pour chaque direction du regard D1, D2 à la première distance de travail déterminée à l'étape b), l'intersection entre cette direction du regard et ladite surface, de manière à établir une cartographie de ces points d'intersection I1, I2, I3, I4 sur cette surface.

La position de la surface considérée, ici le plan moyen du cercle de la monture, étant connue par rapport à la tête du porteur, et la position de la tête du porteur étant connue dans le référentiel du dispositif de capture d'image grâce au système de repérage 40, l'unité de traitement informatique en déduit la position de ce plan moyen PM dans le référentiel du dispositif de capture d'image 21.

Puis l'unité de traitement d'image détermine par le calcul l'intersection de ce plan moyen PM et de la direction de regard D1, D2 à la première distance de travail.

A cet effet, elle détermine les coordonnées (x, y) du point d'intersection I1, I2, I3, I4 de la direction de regard D1, D2 et du plan moyen PM du cercle de la monture 10 dans un repère orthonormé (X, Y) de ce plan moyen PM.

Ici, dans ce repère orthonormé (X, Y), l'axe Y correspond à la projection de la direction verticale sur le plan moyen PM du cercle de la monture 10. L'axe X est un axe perpendiculaire à l'axe Y, dans ce plan moyen PM.

On peut également utiliser le repère de la norme nommée « Boxing », avec les axes de symétrie de la monture 10 ou du système de repérage 40.

### Etape e)

A l'étape e), l'unité de traitement informatique détermine au moins l'une des grandeurs suivantes :
- une valeur moyenne de l'angle d'abaissement A1, A2 du regard en vision de près,
- les dimensions et la position d'une zone d'usure ZU de la lentille ophtalmique contenant les positions de tous les points d'intersection I1, I2 déterminées,
- une position du barycentre de la zone d'usure ZU,
- la distribution des positions de ces points d'intersection I1, I2 dans cette zone d'usure.

L'angle d'abaissement du regard est défini comme l'angle entre la projection de la direction de regard à la première distance de travail déterminée à l'étape b) et une direction de regard primaire DPR prédéterminée.

Cette direction de regard primaire DPR prédéterminée correspond à la direction de regard du porteur dans des conditions de vision de loin, c'est-à-dire à une deuxième distance de travail correspondant à la vision de loin.

Pour la déterminer, selon un exemple non couvert par l'invention, l'opérateur réalise une étape a') dans laquelle il place le porteur dans une situation dans laquelle il effectue une tâche visuelle à une deuxième distance de travail.

Pour cela, il demande par exemple au porteur de regarder au loin, c'est-à-dire de fixer un point distant d'au moins 5 mètres de ce porteur.

Dans une étape b') on détermine, lors de cette tâche visuelle en vision de loin, au moins une direction du regard du porteur à cette deuxième distance de travail.

De préférence, à l'étape b), on détermine au moins quatre directions de regard dans la première distance de travail. On en déduit quatre points d'intersection I1, I2, I3, I4 (figure 2).

De préférence, au moins deux directions de regard dans la première distance de travail sont espacées d'un angle non nul selon une direction horizontale, qui correspond ici à l'axe X du repère orthonormé du plan moyen PM du cercle de la monture 10. De préférence, au moins deux directions de regard dans la première distance de travail sont espacées d'un angle non nul selon une direction verticale, qui correspond ici à l'axe Y du repère orthonormé du plan moyen PM du cercle de la monture 10.

En outre, lesdites directions de regard dans la première distance de travail correspondent toutes ici à une vision de près du porteur.

De préférence encore, afin d'obtenir des points d'intersection représentant au mieux la zone d'usure ZU, lesdites directions de regard dans la première distance de travail sont espacées d'au moins 5 degrés d'angle dans le référentiel du dispositif de capture d'image.

De préférence, lesdites directions de regard dans la première distance de travail sont espacées d'au moins 15 degrés d'angle pour une tâche visuelle répartie sur une surface mesurant 17 centimètres de largeur sur 24 centimètres de longueur et effectuée à une distance de travail égale à 40 centimètres.

En pratique, on détermine de préférence un nombre de points d'intersection compris entre 2 et 500 points, de préférence entre 50 à 250, de préférence supérieur à 10 points.

Il est ainsi par exemple possible de déterminer une valeur moyenne de l'angle d'abaissement du regard comme étant la moyenne arithmétique des valeurs d'angle d'abaissement du regard déterminée pour chaque direction de regard dans la première distance de travail déterminée à l'étape b).

Une zone d'usure ZU peut être déterminée de manière à englober tous les points d'intersection I1, I2, I3, I4 déterminés.

Il peut s'agir d'une ellipse ou d'un rectangle qui contient tout ou un poucentage des points d'intersection déterminés. De préférence, le contour de la zone d'usure ZU entoure au moins 95% des points d'intersection déterminés.

La distribution F1, F2 (figures 3 et 4) des points d'intersection déterminés à l'étape d) dans cette zone d'usure ZU peut être déterminée par exemple le long de l'axe Y du repère orthonormé (X, Y) du plan moyen PM. Il s'agit alors par exemple de repérer chaque point d'intersection par sa coordonnée le long de cet axe Y et de décompter le nombre de points d'intersection dont la coordonnée correspondante est comprise dans un intervalle de coordonnées donné.

On trace ensuite la courbe donnant le nombre de points d'intersection en fonction de la coordonnée selon l'axe Y pour obtenir la distribution F1, F2 des points d'intersection.

On peut également déterminer une dispersion des points d'intersection.

A cet effet, on détermine les coordonnées (xm, ym) du barycentre des points d'intersection I1, I2, I3, I4 et on calcul la dispersion par rapport à ce barycentre par une formule classique type variance, écart type, erreur type, etc.

En outre, à l'étape e), l'unité de traitement informatique détermine la longueur de progression LP ou, selon un exemple non couvert par l'invention, au moins l'un des paramètres de conception optique suivant : inset E, distance de lecture, ou position de la zone de vision de près.

Cette détermination se fait par exemple en fonction de l'une des grandeurs déterminées définies précédemment.

La longueur de progression LP peut, selon un exemple non couvert par l'invention, être déterminée en fonction de la valeur moyenne de l'angle d'abaissement A1, A2 du regard en vision de près.

La longueur de progression LP est selon l'invention déterminée en fonction de la dimension de la zone d'usure ZU le long de la direction verticale Y contenant les positions d'au moins une partie des points d'intersection dans la zone d'usure le long de cette direction.

Plus la zone d'usure déterminée est grande, plus la longueur de progression déduite est faible.

En outre, la longueur de progression LP déterminée tient également compte de la forme des cercles de la monture choisie.

La hauteur totale du cercle de la monture 10 choisie peut être saisie à la main, déterminée à partir d'une numérisation de la monture ou extraite d'une base de données prédéterminée.

L'étalement vertical de la zone d'usure, c'est-à-dire la dimension de la zone d'usure selon la direction verticale Y de la lentille peut ainsi permettre d'ajuster la longueur de progression LP de manière à remonter la zone de vision de près, de telle sorte que celle-ci soit inclue dans la lentille ophtalmique. Cette correction de la longueur de progression est d'autant plus importante que l'étalement vertical est grand afin de disposer d'une grande zone de vision de près facilement accessible et adaptée au comportement visuel du porteur.

Dans un autre exemple, la position du barycentre de la zone d'usure et l'étalement vertical de la zone d'usure ZU permettent de déterminer la hauteur de la zone de vision de près 12.

La distribution des points d'intersection dans cette zone d'usure peut également permettre de déterminer la longueur de progression déduite.

A titre d'exemple, la méthode selon l'invention peut permettre de choisir entre deux longueurs de progressions égales par exemple à 14 et 18 millimètres.

Par exemple, on utilise la position en x et/ou en y du barycentre de la zone d'usure pour déterminer la position en x et/ou en y du point de référence en vision de près IVP de la lentille progressive.

Le point de référence en vision de loin étant prédéterminé, on peut en déduit la valeur de l'inset E correspondant.

Dans un autre exemple, la limite haute de la zone d'usure est utilisée pour ajuster le profil de progression à 85% d'addition de puissance sphérique, qui correspond au début de la zone de vision de près 12, en modifiant le profil de progression du design de manière à fournir une zone de vision de près 12 cohérente avec le comportement visuel du porteur.

De plus, on peut prévoir d'ajuster la longueur de progression LP de manière à ce que le barycentre de la zone d'usure corresponde à une addition de 100%, c'est-à-dire fasse partie de la zone de vision de près 12.Dans l'exemple de mise en oeuvre décrit ici, la zone d'usure déterminée est située dans la zone de vision de près de la lentille ophtalmique du porteur.

Ainsi, les directions de regard du porteur dans la première distance de travail utilisées pour déterminer le groupe de points d'intersection afin d'en déduire cette zone d'usure sont des directions de regard en vision de près.

Selon un exemple non couvert par l'invention, on peut également réaliser des mesures analogues pour la vision de loin ou la vision intermédiaire du porteur.

Il s'agit alors de déterminer par exemple un groupe de points d'intersection entre des directions de regard en vision de loin. Les directions de regard en vision de loin sont alors espacées d'un angle d'au moins 5 degrés.

Il est entendu que les directions de regard en vision de loin sont espacées d'au moins 5 degrés d'angle des directions de regard en vision de près.

Enfin, le paramètre de conception optique déterminé à l'étape e) peut comprendre une amplitude du déplacement d'au moins un oeil du porteur pendant la tâche de lecture et/ou une amplitude du déplacement de la tête du porteur pendant la tâche de lecture, et/ou un coefficient œil-tête égal au rapport entre l'amplitude du déplacement d'un oeil du porteur selon une direction déterminée et l'amplitude maximale du déplacement de cet oeil pendant la tâche de lecture.

Pour cela, à l'étape a), on place le porteur dans une situation dans laquelle il effectue une tâche de lecture prédéfinie.

Cette tâche de lecture implique la lecture d'au moins un paragraphe comportant plusieurs lignes, par exemple 4 ou 5 lignes, de gauche à droite ou de droite à gauche selon la langue concernée.

On constate que le porteur prend une position plus naturelle au fur et à mesure de la lecture, notamment lorsqu'il change de page.

On utilise donc de préférence un texte comportant plusieurs pages, et on exploite plus particulièrement les mesures correspondant aux dernières pages lues.

On réalise les étapes b), c) et d) telles que décrites précédemment, de manière à déterminer une zone d'usure en vision de près d'au moins une des lentilles ophtalmiques destinées à la monture choisie par le porteur.

A l'étape e), on détermine les dimensions de ladite zone d'usure, en particulier une largeur de la zone d'usure mesurée selon une direction horizontale et une hauteur de la zone d'usure mesurée selon une direction verticale de la lentille.

On en déduit l'amplitude angulaire du déplacement des yeux du porteur lors de la tâche de lecture, selon la direction horizontale et selon la direction verticale.

De préférence, la mesure est réalisée pour chacun des yeux du porteur de manière à déterminer les dimensions des zones d'usure des lentilles ophtalmiques gauches et droites destinées à équiper ce porteur.

Connaissant la position de chaque centre de rotation de l'œil par rapport à la lentille ophtalmique, on en déduit les amplitudes angulaires de déplacement de l'œil droit et de l'œil gauche. On peut par exemple à cet effet considérer que le centre de rotation de l'œil est situé en moyenne à 27 millimètres de la face arrière de la lentille ophtalmique.

Connaissant les dimensions du texte affiché sur le support et lu par le porteur, ainsi que la distance de lecture du porteur, c'est-à-dire la distance entre les yeux du porteur et ce texte, on détermine de préférence l'amplitude angulaire maximale du déplacement de chaque oeil comme étant l'étendue angulaire du texte vu par le porteur, selon la direction horizontale ou verticale considérée.

La différence entre l'amplitude angulaire maximale de déplacement des yeux et l'amplitude angulaire du déplacement effectif des yeux mesuré correspond en fait à l'amplitude du déplacement de la tête du porteur pendant la lecture. Il est donc également possible de déduire ce paramètre de la zone d'usure déterminée.

En divisant l'amplitude angulaire du mouvement de chaque oeil par l'amplitude angulaire maximale du déplacement de chaque oeil, on en déduit un coefficient appelé coefficient œil-tête caractéristique du comportement du porteur lors d'une tâche de lecture.

Ce coefficient quantifie la propension du porteur à bouger les yeux ou la tête lors de la tâche de lecture.

Il est important de le prendre en compte en vue de la conception optique de la lentille, notamment pour la détermination de la zone de vision de près de la lentille.

Un porteur ayant un coefficient œil-tête proche de 1 sera d'autant plus à l'aise en vision de près si la zone de vision de près est large.

On peut donc prévoir de déterminer, à l'étape e), la longueur de progression et/ou la position et les dimensions de la zone de vision de près en fonction de ce coefficient.

Il est également possible de déterminer la moyenne de l'amplitude angulaire du déplacement de l'œil comme étant la moyenne des déplacements angulaire de l'œil gauche et de l'œil droit du porteur. On peut alors en déduire un coefficient œil-tête moyen.

En variante, on peut également suivre le mouvement de la tête grâce à au dispositif de capture d'image et déterminer directement l'amplitude angulaire du déplacement de la tête pendant la lecture.

Cette prise en compte du comportement œil/tête du porteur permet d'assurer un confort visuel optimal pour ce porteur.

On a décrit ici un exemple particulier de dispositif pour la mise en oeuvre de la méthode selon l'invention. Plus généralement, un tel dispositif de mesure comportant :
- des moyens de capture d'image,
- des moyens d'affichage d'une cible en mouvement dont la position est connue dans un référentiel lié au dispositif de capture d'image, programmés pour enregistrer, à chaque capture d'image, la position correspondante de la cible sur les moyens d'affichage ou programmés pour déclencher une capture d'image lorsque la cible présente une position de déclenchement prédéterminée sur les moyens d'affichage, peut permettre la mise en oeuvre de cette méthode.

Ce dispositif de mesure comporte notamment des moyens pour synchroniser l'affichage de la cible et la capture d'une image.

## Revendications

1. Méthode de détermination d'une longueur de progression (LP, LP1, LP2) d'une lentille ophtalmique progressive destinée à équiper une monture (10) choisie par un porteur, en fonction du comportement visuel de celui-ci, comprenant les étapes suivantes :
a) on place le porteur dans une situation dans laquelle il effectue une tâche visuelle à une première distance de travail en vision de près,
b) on détermine, lors de cette tâche visuelle, au moins deux directions du regard (D1, D2) du porteur à cette première distance de travail dans un référentiel de la tête du porteur,
c) on détermine une position d'une surface (PM) ou d'une ligne liée à ladite monture (10) ou à une lentille ophtalmique destinée à équiper ladite monture (10) dans ce référentiel de la tête du porteur,
d) on détermine, pour chaque direction du regard (D1, D2) à la première distance de travail déterminée à l'étape b), l'intersection entre cette direction du regard (D1, D2) à la première distance de travail et ladite surface (PM) ou ladite ligne, de manière à établir une cartographie de ces points d'intersection (I1, I2, I3, I4) avec ladite surface (PM) ou ladite ligne,
e) on déduit de cette cartographie ladite longueur de progression (LP, LP1, LP2), en déterminant les dimensions d'une zone d'usure (ZU), le long d'une direction verticale (Y), de la lentille ophtalmique (30) contenant positions d'au moins une partie des points d'intersection (I1, I2, I3, I4) déterminés de sorte que plus la zone d'usure déterminée est grande, plus la longueur de progression déduite est faible.

2. Méthode selon la revendication 1, selon laquelle, à l'étape a), la première distance de travail est une distance de vision de près et le porteur effectue une tâche de lecture ou d'écriture ou une tâche interactive ou une tâche d'observation.

3. Méthode selon l'une des revendications précédentes, selon laquelle, à l'étape c), ladite surface est l'une des surfaces suivantes : surface moyenne de la lentille ophtalmique, plan moyen (PM) d'un cercle de la monture, plan moyen des cercles de la monture, face avant ou arrière de la lentille ophtalmique.

4. Méthode selon l'une des revendications précédentes, selon laquelle, à l'étape a), le porteur suit des yeux une cible (C1, C2) dont la position est connue dans un référentiel lié à un dispositif de capture d'image (21) et à l'étape b), on réalise, à l'aide de ce dispositif de capture d'image (21), une capture d'image de la tête du porteur pour différentes positions de la cible correspondant aux différentes directions de regard (D1, D2) à la première distance de travail.

5. Méthode selon la revendication 4, selon laquelle, à l'étape b), pour chaque image capturée, on détermine la position du centre de rotation (CRO) d'au moins un oeil du porteur dans le référentiel lié à la tête du porteur et on en déduit chaque direction du regard D1, D2) à la première distance de travail comme étant la droite reliant ce centre de rotation (CRO) avec la cible (C1, C2) dans sa position correspondante.

6. Méthode selon la revendication 4, selon laquelle, à l'étape b), pour chaque image capturée alors que le porteur est muni de la monture équipée de lentilles ophtalmiques, on détermine la position du centre de rotation d'au moins un oeil du porteur dans le référentiel lié à la tête du porteur et on en déduit la direction du regard à la première distance de travail comme étant le chemin optique reliant le centre de rotation de l'œil et la cible en tenant compte de la déviation prismatique de ladite lentille ophtalmique.

7. Méthode selon l'une des revendications 5 et 6, selon laquelle, à l'étape b), on estime approximativement la position moyenne du centre de rotation de l'œil du porteur dans le référentiel lié à la tête du porteur.

8. Méthode selon l'une des revendications 5 et 6, selon laquelle, à l'étape b), on mesure une position de référence des centres de rotation des yeux dans le référentiel lié à la tête du porteur dans une étape préliminaire et on enregistre cette position de référence.

9. Méthode selon la revendication 4, selon laquelle, à l'étape b), on capture chaque image du porteur lorsque la cible se trouve sur l'axe optique de du dispositif de capture d'image.

10. Méthode selon la revendication 9, selon laquelle, à l'étape d), on détermine l'intersection entre la direction du regard à la première distance de travail et ladite surface en mesurant directement sur l'image capturée la position de l'image de la pupille de l'oeil du porteur sur l'image de la lentille ophtalmique.

11. Méthode selon l'une des revendications 1 à 10, selon laquelle, à l'étape a), le porteur est muni de la monture de lunettes choisie et, à l'étape c), on détermine la position de ladite surface ou de ladite ligne dans le référentiel de la tête du porteur à partir d'au moins une image capturée de la tête du porteur pendant ladite tâche visuelle.

12. Méthode selon l'une des revendications 1 à 10, selon laquelle, à l'étape c), on détermine la position de ladite surface ou de ladite ligne dans le référentiel de la tête du porteur à partir d'une base de données.

13. Méthode selon l'une des revendications précédentes, selon laquelle, au moins deux desdites directions de regard (D1, D2) à la première distance de travail sont espacées d'au moins 5 degrés d'angle en projection dans un plan horizontal.

14. Méthode selon l'une des revendications précédentes, selon laquelle, à l'étape a), le porteur effectue une tâche visuelle de lecture en vision de près, et, à l'étape e), on déduit de la cartographie déterminée à l'étape d) une zone d'usure en vision de près de la lentille, et/ou une amplitude du déplacement d'au moins un oeil du porteur pendant la tâche de lecture et/ou une amplitude du déplacement de la tête du porteur pendant la tâche de lecture, et/ou un coefficient oeil-tête égal au rapport entre l'amplitude du déplacement d'un oeil du porteur selon une direction déterminée et l'amplitude maximale du déplacement de cet oeil pendant la tâche de lecture.

15. Système comprenant un dispositif de mesure et une unité de traitement informatique adaptés pour la mise en oeuvre de la méthode selon l'une des revendications précédentes, ledit dispositif de mesure comportant
- des moyens de capture d'image (21),
- des moyens d'affichage (20) d'une cible (C1, C2) en mouvement dont la position est connue dans un référentiel lié au dispositif de capture d'image (21),
programmés pour déclencher une capture d'image lorsque la cible (C1, C2) présente une position de déclenchement prédéterminée sur les moyens d'affichage (20).

## Patentansprüche

1. Verfahren zur Bestimmung einer Progressionslänge (LP, LP1, LP2) einer ophthalmischen Gleitsichtlinse, die eine von einem Träger gewählte Fassung (10) bestücken soll, in Abhängigkeit von dessen Sehverhalten, umfassend die folgenden Schritte:
a) man versetzt den Träger in eine Situation, in der er eine Sehaufgabe in einem ersten Arbeitsabstand in Nahsicht ausführt,
b) man bestimmt bei dieser Sehaufgabe mindestens zwei Blickrichtungen (D1, D2) des Trägers in diesem ersten Arbeitsabstand in einem Bezugssystem des Kopfes des Trägers,
c) man bestimmt eine Position einer Fläche (PM) oder einer Linie, die mit der Fassung (10) oder mit einer ophthalmischen Linse, die die Fassung (10) bestücken soll, verbunden ist, in diesem Bezugssystem des Kopfes des Trägers,
d) man bestimmt für jede Blickrichtung (D1, D2) in dem ersten Arbeitsabstand, die im Schritt b) bestimmt wurde, den Schnittpunkt zwischen dieser Blickrichtung (D1, D2) in dem ersten Arbeitsabstand und der Fläche (PM) oder der Linie, so dass eine Kartografie dieser Schnittpunkte (11, 12, 13, 14) mit der Fläche (PM) oder der Linie erstellt wird,
e) man leitet aus dieser Kartografie die Progressionslänge (LP, LP1, LP2) ab, indem man die Abmessungen einer Abnutzungszone (ZU) entlang einer vertikalen Richtung (Y) der ophthalmischen Linse (30) bestimmt, der die Positionen mindestens eines Teils der bestimmten Schnittpunkte (11, 12, 13, 14) enthält, so dass die abgeleitete Progressionslänge umso geringer ist, je größer die bestimmte Abnutzungszone ist.

2. Verfahren nach Anspruch 1, bei dem im Schritt a) der erste Arbeitsabstand ein Nahsichtabstand ist und der Träger eine Lese- oder Schreibaufgabe oder eine interaktive Aufgabe oder eine Beobachtungsaufgabe ausführt.

3. Verfahren nach einem der vorhergehenden Ansprüche, bei dem im Schritt c) die Fläche eine der folgenden Flächen ist: mittlere Fläche der ophthalmischen Linse, Mittelebene (PM) eines Rahmens der Fassung, Mittelebene der Rahmen der Fassung, Vorder- oder Rückseite der ophthalmischen Linse.

4. Verfahren nach einem der vorhergehenden Ansprüche, bei dem der Träger im Schritt a) mit den Augen einem Ziel (C1, C2) folgt, dessen Position in einem Bezugssystem, das mit einer Bildaufnahmevorrichtung (21) verbunden ist, bekannt ist, und man im Schritt b) mithilfe dieser Bildaufnahmevorrichtung (21) eine Bildaufnahme des Kopfes des Trägers für verschiedene Positionen des Ziels ausführt, die den verschiedenen Blickrichtungen (D1, D2) in dem ersten Arbeitsabstand entsprechen.

5. Verfahren nach Anspruch 4, bei dem man im Schritt b) für jedes aufgenommene Bild die Position des Rotationsmittelpunkts (CRC) mindestens eines Auges des Trägers in dem Bezugssystem, das mit dem Kopf des Trägers verbunden ist, bestimmt und man daraus jede Blickrichtung (D1, D2) in dem ersten Abstandsabstand als die Gerade bestimmt, die diesen Rotationsmittelpunkt (CRC) mit dem Ziel (C1, C2) in seiner entsprechenden Position verbindet.

6. Verfahren nach Anspruch 4, bei dem man im Schritt b) für jedes aufgenommene Bild, während der Träger mit der mit ophthalmischen Linsen bestückten Fassung versehen ist, die Position des Rotationsmittelpunkts mindestens eines Auges des Trägers in dem Bezugssystem, das mit dem Kopf des Trägers verbunden ist, bestimmt und man daraus die Blickrichtung in dem ersten Abstandsabstand als den optischen Pfad bestimmt, der den Rotationsmittelpunkt des Auges und das Ziel verbindet, unter Berücksichtigung der prismatischen Ablenkung der ophthalmischen Linse.

7. Verfahren nach einem der Ansprüche 5 und 6, bei dem man im Schritt b) die mittlere Position des Rotationsmittelpunkts des Auges des Trägers in dem Bezugssystem, das mit dem Kopf des Trägers verbunden ist, näherungsweise schätzt.

8. Verfahren nach einem der Ansprüche 5 und 6, bei dem man im Schritt b) eine Bezugsposition der Rotationsmittelpunkte der Augen in dem Bezugssystem, das mit dem Kopf des Trägers verbunden ist, in einem vorbereitenden Schritt misst und man diese Bezugsposition aufzeichnet.

9. Verfahren nach Anspruch 4, bei dem man im Schritt b) jedes Bild des Trägers aufnimmt, wenn sich das Ziel auf der optischen Achse der Bildaufnahmevorrichtung befindet.

10. Verfahren nach Anspruch 9, bei dem man im Schritt d) den Schnittpunkt zwischen der Blickrichtung in dem ersten Arbeitsabstand und der Fläche bestimmt, indem man direkt auf dem aufgenommenen Bild die Position des Bildes der Pupille des Auges des Trägers auf dem Bild der ophthalmischen Linse misst.

11. Verfahren nach einem der Ansprüche 1 bis 10, bei dem der Träger im Schritt a) mit der gewählten Brillenfassung versehen ist und bei dem man im Schritt c) die Position der Fläche oder der Linie in dem Bezugssystem des Kopfes des Trägers anhand mindestens eines Bildes bestimmt, das vom Kopf des Trägers während der Sehaufgabe aufgenommen wurde.

12. Verfahren nach einem der Ansprüche 1 bis 10, bei dem man im Schritt c) die Position der Fläche oder der Linie in dem Bezugssystem des Kopfes des Trägers anhand einer Datenbank bestimmt.

13. Verfahren nach einem der vorhergehenden Ansprüche, bei dem mindestens zwei der Blickrichtungen (D1, D2) in dem ersten Arbeitsabstand um mindestens 5 Grad Projektionswinkel in einer horizontalen Ebene beabstandet sind.

14. Verfahren nach einem der vorhergehenden Ansprüche, bei dem der Träger im Schritt a) eine Lesesehaufgabe in Nahsicht ausführt und man im Schritt e) aus der im Schritt d) bestimmten Kartografie einer Nahsicht-Abnutzungszone der Linse und/oder eine Amplitude der Bewegung mindestens eines Auges des Trägers während der Leseaufgabe und/oder eine Amplitude der Bewegung des Kopfes des Trägers während der Leseaufgabe und/oder einen Auge-Kopf-Koeffizienten gleich dem Verhältnis zwischen der Amplitude der Bewegung eines Auges des Trägers entlang einer bestimmten Richtung und der maximalen Amplitude der Bewegung dieses Auges während der Leseaufgabe ableitet.

15. System, umfassend eine Messvorrichtung und eine informationstechnische Verarbeitungseinheit, die zur Umsetzung des Verfahrens nach einem der vorhergehenden Ansprüche geeignet sind, wobei die Messvorrichtung beinhaltet
- Bildaufnahmeeinrichtungen (21),
- Anzeigeeinrichtungen (20) zum Anzeigen eines Ziels (C1, C2) in Bewegung, dessen Position in einem Bezugssystem, das mit der Bildaufnahmevorrichtung (21) verbunden ist, bekannt ist,
die dazu programmiert sind, eine Bildaufnahme auszulösen, wenn das Ziel (C1, C2) eine vorbestimmte Auslöseposition auf den Anzeigeeinrichtungen (20) aufweist.

## Claims

1. Method for determining a progression length (LP, LP1, LP2) of a progressive ophthalmic lens intended to equip a frame (10) chosen by a wearer, depending on the visual behaviour of the latter, comprising the following steps:
a) placing the wearer in a situation in which he performs a visual task at a first near-vision working distance;
b) determining, during this visual task, at least two directions (D1, D2) of the gaze of the wearer at this first working distance in a frame of reference of the head of the wearer;
c) determining a position of a surface (PM) or of a line associated with said frame (10) or with an ophthalmic lens intended to equip said frame (10) in this frame of reference of the head of the wearer;
d) determining, for each direction (D1, D2) of the gaze at the first working distance determined in step b), the intersection between this direction (D1, D2) of the gaze at the first working distance and said surface (PM) or said line, so as to establish a map of these points of intersection (11, 12, 13, 14) with said surface (PM) or said line; and
e) deducing from this map said progression length (LP, LP1, LP2), by determining the dimensions of a zone of use (ZU), along a vertical direction (Y), of the ophthalmic lens (30) containing the positions of at least some of the determined points of intersection (11, 12, 13, 14) so that the larger the determined zone of use, the shorter the deduced progression length.

2. Method according to Claim 1, wherein, in step a), the first working distance is a near-vision distance and the wearer performs a reading or writing task or an interactive task or an observation task.

3. Method according to one of the preceding claims, wherein, in step c), said surface is one of the following surfaces: mean surface of the ophthalmic lens, mean plane (PM) of a rim of the frame, mean plane of the rims of the frame, back or front face of the ophthalmic lens.

4. Method according to one of the preceding claims, wherein, in step a), the wearer follows with his eyes a target (C1, C2), the position of which is known in a frame of reference associated with an image-capturing device (21) and in step b), using this image-capturing device (21), an image of the head of the wearer is captured for various positions of the target corresponding to the various gaze directions (D1, D2) at the first working distance.

5. Method according to Claim 4, wherein, in step b), for each captured image, the position of the rotation centre (CRO) of at least one eye of the wearer is determined in the frame of reference associated with the head of the wearer and therefrom each direction (D1, D2) of the gaze at the first working distance is deduced as being the straight line connecting this rotation centre (CRO) with the target (C1, C2) in its corresponding position.

6. Method according to Claim 4, wherein, in step b), for each image captured while the wearer is equipped with the frame fitted with ophthalmic lenses, the position of the rotation centre of at least one eye of the wearer is determined in the frame of reference associated with the head of the wearer and therefrom the direction of the gaze at the first working distance is deduced as being the optical path connecting the rotation centre of the eye and the target, while taking into account the prismatic deviation of said ophthalmic lens.

7. Method according to one of Claims 5 and 6, wherein, in step b), the mean position of the rotation centre of the eye of the wearer is approximately estimated in the frame of reference associated with the head of the wearer.

8. Method according to one of Claims 5 and 6, wherein, in step b), a reference position of the rotation centres of the eyes is measured in the frame of reference associated with the head of the wearer in a preliminary step and this reference position is recorded.

9. Method according to Claim 4, wherein, in step b), each image of the wearer is captured when the target is found on the optical axis of the image-capturing device.

10. Method according to Claim 9, wherein, in step d), the intersection between the direction of the gaze at the first working distance and said surface is determined by directly measuring in the captured image the position of the image of the pupil of the eye of the wearer in the image of the ophthalmic lens.

11. Method according to one of Claims 1 to 10, wherein, in step a), the wearer is equipped with the chosen spectacle frame and, in step c), the position of said surface or of said line is determined in the frame of reference of the head of the wearer from at least one captured image of the head of the wearer during said visual task.

12. Method according to one of Claims 1 to 10, wherein, in step c), the position of said surface or of said line is determined in the frame of reference of the head of the wearer from a database.

13. Method according to one of the preceding claims, wherein at least two said gaze directions (D1, D2) at the first working distance are spaced apart by at least 5 degrees of angle in projection in a horizontal plane.

14. Method according to one of the preceding claims, wherein, in step a), the wearer performs a near-vision visual reading task, and in step e), from the map determined in step d), a zone of near-vision use of the lens, and/or an amplitude of the movement of at least one eye of the wearer during the reading task and/or an amplitude of the movement of the head of the wearer during the reading task, and/or an eye-head coefficient equal to the ratio of the amplitude of the movement of an eye of the wearer in a determined direction to the maximum amplitude of the movement of this eye during the reading task are/is deduced.

15. System comprising a measuring device and information-processing unit suitable for implementing the method as claimed in one of the preceding claims, said measuring device including
- image-capturing means (21);
- means (20) for displaying a moving target (C1, C2), the position of which is known in a frame of reference associated with the image-capturing device (21) ;
which means are programmed to trigger an image capture when the target (C1, C2) has a predetermined triggering position on the displaying means (20).
